# EUROPÄISCHE PATENTSCHRIFT

(11) **EP 1 648 568 B1**
(45) Veröffentlichungstag und Bekanntmachung des Hinweises auf die Patenterteilung: **22.11.2006**
(21) Anmeldenummer: 04729068.9
(22) Anmeldetag: 23.04.2004
(51) Int. Cl.: A61Q 19/02, A61K 8/97, A61K 8/34, A61K 8/39, A61K 8/49, A61K 8/99

(54) **KOSMETIKUM ZUR DEPIGMENTIERUNG**
COSMETIC FOR DEPIGMENTATION
PRODUIT COSMETIQUE DEPIGMENTANT

(30) Priorität: 24.04.2003 DE 10319836; 01.03.2004 DE 102004010645
(43) Veröffentlichungstag der Anmeldung: 26.04.2006
(73) Patentinhaber: Coty B.V., 2031 CC Haarlem (NL)
(72) Erfinder: GOLZ-BERNER, Karin, MC-98000 Monaco (MC); ZASTROW, Leonhard, MC-98000 Monaco (MC); Bernini, Dorothee, MC-98000 Monaco (MC)
(74) Vertreter: Ziebig, Marlene
(86) Internationale Anmeldenummer: PCT/EP2004/004358
(87) Internationale Veröffentlichungsnummer: WO 2004/093839

(56) Entgegenhaltungen:
- EP-A- 1 281 396
- US-A- 6 010 701
- US-B1- 6 436 378
- PATENT ABSTRACTS OF JAPAN Bd. 2002, Nr. 11, 6. November 2002 (2002-11-06) & JP 2002 205933 A (ICHIMARU PHARCOS CO LTD), 23. Juli 2002 (2002-07-23)
- DATABASE BIOSIS [Online] BIOSCIENCES INFORMATION SERVICE, PHILADELPHIA, PA, US; Februar 2003 (2003-02), FU GUO-QIANG ET AL: "Inhibitory effects of ethanolic extracts from 196 kind of Chinese herbs on tyrosinase." XP002292067 Database accession no. PREV200300234856 & ZHONGHUA PIFUKE ZAZHI, Bd. 36, Nr. 2, Februar 2003 (2003-02), Seiten 103-106, ISSN: 0412-4030
- PATENT ABSTRACTS OF JAPAN Bd. 2000, Nr. 05, 14. September 2000 (2000-09-14) & JP 2000 060527 A (KAWANO MERIKURON:KK), 29. Februar 2000 (2000-02-29)
- [Online] XP002292064 Cosmetochem - A natural touch in cosmetology: Botanical extract Gefunden im Internet: URL:http://www.cosmetochem.ch/>
- [Online] XP002292065 Cosmetochem - A natural touch in cosmetology Gefunden im Internet: URL:http://www.cosmetochem.ch/>
- YOSHIKAWA M ET AL: "NOVEL INDOLE S,O-BISDESMOSIDE, CALANTHOSIDE, THE PRECURSOR GLYCOSIDE OF TRYPTANTHRIN, INDIRUBIN, AND ISATIN, WITH INCREASING SKIN BLOOD FLOW PROMOTING EFFECTS, FROM TWO CALANTHE SPECIES (ORCHIDACEAE" CHEMICAL AND PHARMACEUTICAL BULLETIN, PHARMACEUTICAL SOCIETY OF JAPAN. TOKYO, JP, Bd. 46, Nr. 5, 1998, Seiten 886-888, XP000872921 ISSN: 0009-2363
- [Online] XP002292066 Gattefossé - with in vitro substantiated claims Gefunden im Internet: URL:http://www.gattefosse.com/cosmetic/mai nvitext.htm>

## Beschreibung

Die Erfindung betrifft ein Kosmetikum mit depigmentierender und dadurch hautaufhellender Wirkung.

Es sind bereits hautaufhellende Mittel und Methoden bekannt, wie ein Verfahren zur Hautaufhellung mittels eines Gemisches aus einem antimikrobiellen Mittel, einer α- bzw. β-Hydroxysäure wie Glycolsäure oder Milchsäure bzw. Salicylsäure und einem pharmazeutisch annehmbaren Träger (WO 01/70189).

Aus der WO98/58628 sind zur Hautaufhellung Kojisäure, Ascorbinsäure, Bärentraubenextrakt, Maulbeerbaumextrakt, Süßholzextrakt, Arbutin und Gemische davon bekannt, die in der WO02/36090 auch in Form eines kosmetischen Pflasters angewendet werden.

In der EP 909161 werden substituierte Diphenole in einer Wasser-in-Silicon-Gelzusammensetzung als Hautaufhellungsmittel beschrieben. Die Wirkung dieser Mittel ist nicht immer befriedigend.

Der Erfindung liegt die Aufgabe zugrunde, neue Kosmetika bereitzustellen, die eine hautaufhellende oder depigmentierende Wirksamkeit zeigen.

Erfindungsgemäß enthält das neue Kosmetikum einen Extrakt der oberirdischen Teile einer weißen Orchidee, insbesondere der Orchidee Phalaenopsis amabilis sowie übliche kosmetischen Trägerstoffe und/oder Hilfsstoffe und/oder weitere Wirkstoffe oder Gemische davon.

Das erfindungsgemäße Kosmetikum enthält insbesondere den Extrakt einer ganz spezifischen Orchideenspezies. Es sind weltweit etwa 25.000 bis 30.000 Orchideenarten bekannt. Allein die Art Phalaenopsis umfaßt etwa 40 Spezies in mehr als hundert verschiedenen Farben. Diese Art ist im tropischen Asien bis Südindien beheimatet. Die weiße Orchidee Phalaenopsis amabilis wird in Burma, Buthan und im südlichen Indien gefunden.

Es wird ein Extrakt der oberirdischen Teile dieser Pflanze, d.h. der Blätter, Blüten und Stengel eingesetzt. Dieser Extrakt kann ein wäßriger oder alkoholischer oder wäßrig/alkoholischer Extrakt sein. Als Alkohole können niedere einwertige Alkohole wie Ethanol, Propanol, Isopropanol oder Gemische davon eingesetzt werden, oder es werden mehrwertige Alkohole wie z.B Propylenglycol eingesetzt. Die Extraktionstemperatur liegt im Bereich von 10 bis 40 °C. Der erhaltene Extrakt kann in flüssiger oder - nach Sprüh- oder Gefriertrocknung beispielsweise - auch in fester Form in die kosmetische Formulierung eingebracht werden. Bei Einbringen in fester Form sind Puderteilchen mit Teilchengrößen im Bereich von 0,1 bis 100 µm bevorzugt.

Das erfindungsgemäße Kosmetikum zeigt eine keratinolytische Wirksamkeit auf pigmentierte Epidermalzellen und führt somit zum regelrechten Abblättern (exfoliation) dieser pigmentierten Zellen. Darüber hinaus wird vermutlich das Enzym Tyrosinase inhibiert, wodurch die Melaninbildung verhindert wird, da der Teilschritt von Tyrosin zu DOPA auf dem Wege zum Melanin durch Tyrosinase ermöglicht wird. Es wurde gefunden, daß bereits 0,01 Gew-% Extrakt zu einer deutlichen Inhibierung der Melaninbildung führen.

Die Extrakte können sowohl in verkapselter Form als auch unverkapselt in die Formulierung eingebracht werden. Als Verkapselungsmaterial sind verschieden Arten von Liposomen oder andere bekannte Mikrokapseln wie solche aus Lecithin (Phospholipide), Carboxymethycellulose und anderen Materialien einsetzbar.

Der Anteil des Orchideenextraktes von Phalaenopsis amabilis liegt vorteilhaft im Bereich von 0,005 bis 5 Gew-%, wobei Anteile von 0,01 bis 1 Gew-% besonders bevorzugt sind. Es wurde gefunden, daß die Tyrosinase-Aktivität bereits mit 0,03 Gew-% Extrakt um etwa 30 % und mit 0,05 Gew-% Extrakt um 40 % inhibiert wurde gegenüber einem Kontrolltest. Damit konnte bereits durch geringe Konzentrationen des Weiße Orchidee-Extraktes die Wirksamkeit von Tyrosinase auf 60% beschränkt werden.

Der erfindungsgemäße Extrakt kann mit anderen hautaufhellenden Substanzen oder Substanzgemischen vermischt sein, insbesondere mit Pflanzenextrakten wie beispielsweise 0,05 bis 0,3 Gew-% eines Extraktgemisches von Süßholz (Glycyrrhiza glabra) und Aspergillusferment (Verhältnis 30:1 bis 5:1) und 0,001 bis 0,1 Gew-% eines wäßrigen oder wäßrig-alkoholischen Extraktes von Tragant (Astragalus), wobei die Prozentangaben jeweils auf das Gesamtgewicht des Kosmetikums bezogen sind. Die hautaufhellende Wirkung wird dadurch nochmals verstärkt.

Weiterhin ist der Zusatz anderer Pflanzenextrakte, wie zum Beispiel Kiwi-Extrakten in Konzentrationen von 0,5 bis 5 Gew-% eine die Wirksamkeit des Kosmetikums fördernde Maßnahme.

Das Kosmetikum kann weitere Wirkstoffe enthalten. Zu den kosmetischen Wirkstoffen gehören z. B. anorganische und organische Lichtschutzmittel, Radikalfänger, Feuchthaltemittel, Erweichungsmittel, Vitamine, Enzyme, andere pflanzliche Wirkstoffe, Polymere, Antioxidationsmittel, entzündungswidrige natürliche Wirkstoffe, mit Sauerstoff beladene asymmetrische lamellare Aggregate gemäß WO 94/00109; Aufschlußprodukte von Hefen oder pflanzlichen Stoffen, hergestellt durch ein schonendes Ultraschall-Aufschlußverfahren gemäß WO 94/13783, Kaolin sowie mit SiO₂ modifiziertes Kaolin gemäß WO94/17588.

Zu Antioxidationsmitteln gehören Vitamine wie Vitamin C und Derivate davon, beispielsweise Ascorbylacetate, -phosphate und -palmitate; Vitamin A und Derivate davon; Folsäure und deren Derivate, Vitamin E und deren Derivate, wie Tocopherylacetat; Flavone oder Flavonoide; Aminosäuren, wie Histidin, Glycin, Tyrosin, Tryptophan und Derivate davon; Harnsäure und Derivate davon.

Als Erweichungsmittel können normalerweise eine Vielzahl von Verbindungen eingesetzt werden, wie Stearylalkohol, Glycerylmonoricinoleat, Glycerylmonostearat, Propan-1,2-diol, Butan-1,3-diol, Cetylalkohol, Isohexadecan, Isopropylisostearat, Stearinsäure, Isobutylpalmitat, Oleylalkohol, Isopropyllaurat, Decyloleat, Octadecan-2-ol, Isocetylalkohol, Cetylpalmitat, Siliconöle wie Dimethylpolysiloxan, Isopropylmyristat, Isopropylpalmitat, Polyethylenglycol, Lanolin, Kakaobutter, pflanzliche Öle wie Maisöl, Baumwollsamenöl, Olivenöl, mineralische Öle, Butylmyristat, Palmitinsäure usw.

Es ist weiterhin vorteilhaft, den erfindungsgemäßen Zusammensetzungen entsprechende wasser- und/oder öllösliche UVA- oder UVB-Filter oder beide zuzusetzen. Zu vorteilhaften öllöslichen UVB-Filtern gehören 4-Aminobenzoesäure-Derivate wie der 4-(Dimethylamino)-benzoesäure-(2-ethylhexyl)ester; Ester der Zimtsäure wie der 4-Methoxyzimtsäure(2-ethylhexyl)ester, Benzophenon-Derivate wie 2-Hydroxy-4-methoxybenzophenon; 3-Benzylidencampher-Derivate wie 3-Benzylidencampher.

Bevorzugte öllösliche UV-Filter sind Benzophenone-3, Butyl-Methoxybenzoylmethane, Isoamyl p-Methoxycinnamate, Ethylhexyl Methoxycinnamate, Octyl Methoxycinnamate, Octyl Salicylate, 4-Methylbenzylidene Camphor, Homosalate und Octyl Dimethyl PABA.

Wasserlösliche UVB-Filter sind z.B. Sulfonsäurederivate von Benzophenon oder von 3-Benzylidencampher oder Salze wie das Na- oder K-Salz der 2-Phenylbenzimidazol-5-sulfonsäure oder Salicylsäurederivate wie Ethylhexyl Salicylate.

Zu UVA-Filtern gehören Dibenzoylmethan-Derivate wie 1-Phenyl-4-(4'-isopropylphenyl)propan-1,3-dion und Bis-Ethylhexyloxyphenol/Methoxyphenyl Triazine.

Ein weiterer besonders wirksamer Zusatz für das erfindungsgemäße Kosmetikum ist eine Wirkstoffzubereitung mit hohem Radikalschutzfaktor mit einem Gehalt an einem durch Extraktion der Rinde von Quebracho blanco und nachfolgender enzymatischer Hydrolyse gewonnenem Produkt, das wenigstens 90 Gew- % Proanthocyanidin-Oligomere und höchstens 10 Gew-% Gallussäure enthält, in Mikrokapseln, sowie einem durch Extraktion gewonnenen Seidenraupenextrakt, der das Peptid Cecropine, Aminosäuren und ein Vitamingemisch enthält, und einem nichtionischen, kationischen oder anionischen Hydro-Gel oder Gemisch von Hydro-Gelen, und einem oder mehreren Phospholipiden, und Wasser.

Dieses Produkt, z.B. der Wirkstoffkomplex gemäß Beispiel 1 oder 2 der WO99/66881, oder in einer Form, die zusätzlich Superoxiddismutase und Cyclodextrine enthält, z.B. gemäß Beispiel 1 der WO 01/26617, kann die Wirkung der erfindungsgemäßen Formulierung noch weiter verbessern.

Das erfindungsgemäße Kosmetikum enthält weiterhin kosmetische Hilfs- und Trägerstoffe, wie sie üblicherweise in solchen Zubereitungen verwendet werden, z.B. Wasser, Konservierungsmittel, Farbstoffe, Verdikungsmittel, Duftstoffe, Alkohole, Polyole, Ester, Elektrolyte, Gelbildner, polare und unpolare Öle, Polymere, Copolymere, Emulgatoren, Stabilisatoren. Auch geringe Mengen an Pigmenten, wie Eisenoxide oder Titandioxid, können enthalten sein.

Die für die Erfindung eingesetzten Öle können übliche kosmetische Öle sein, wie ein Mineralöl; hydriertes Polyisobuten; synthetisches oder aus Naturprodukten hergestelltes Squalan; kosmetische Ester oder Ether, die verzweigt oder unverzweigt, gesättigt oder ungesättigt sein können; pflanzliche Öle; Siliconöle oder Gemische zweier oder mehrerer davon.

Besonders geeignete Öle sind beispielsweise Mineralöle, Hydrogenated Polyisobuten, Polyisopren, Squalane, Tridecyltrimellitat, Trimethylpropan-triisostearat, Isodecylcitrat, Neopentylglycol-diheptanoat, PPG-15-stearylether, Cyclomethicone sowie pflanzliche Öle, wie Calendulaöl, Jojobaöl, Avocadoöl, Macadamianußöl, Rizinusöl, Weizenkeimöl, Träubenkernöl, Kukuinußöl, Distelöl, Nachtkerzenöl, Safloröl oder ein Gemisch mehrerer davon. Je nachdem welche Öle ausgewählt werden, werden die kosmetischen Eigenschaften beeinflußt, wie Transparenzgrad, Weichheit, Spreitungswirkung.

Als Ester oder Ether sind zum Beispiel geeignet Dipentaerythrityl hexacaprilate/hexacaprate/tridecyl trimellitate/tridecyl stearate/neopentyl glycol dicaprylate dicaprate, Propylene glycol dioctanoate 5, Propylene glycol dicaprylate 2,30 dicaprate, Tridecyl stearate/neopentyl glycol dicaprylate dicaprate/tridecyl trimellitate, Neopentyl glycol dioctanoate, Isopropyle myristate, Diisopropyl dimer dilinoleate, Trimethylpropane triisostearate, Myristyl ether, Stearyl ether, Cetearyl octanoate, Butyl ether, Dicaprylyl ether, PPG1-PEG9 Lauroyl glycol ether, PPG15 Stearyl ether, PPG14 Butyl ether, Fomblin HC25.

Einwertige höhere Alkohole wie Cetearylalkohol oder mehrwertige Alkohole (Polyole) sind ebenfalls mögliche Bestandteile des erfindungsgemäßen Kosmetikums. Dies sind z.B Propylenglycol, Dipropylenglycol, Ethylenglycol, Isoprenglycol, Glycerin, Sorbitol und Gemische davon. Der Anteil des Polyols liegt im Bereich von 0,1 to 40 Gew-%, vorzugsweise von etwa 5% bis etwa 20 Gew-% der Zusammensetzung.

Kosmetische Zusammensetzungen mit der erfindungsgemäßen Wirkstoffzubereitung können als O/W- oder W/O-Emulsionen vorliegen. Geeignete Emulgatoren für O/W- oder W/O-Emulsionen sind bekannt und sind beispielsweise Anlagerungsprodukte von Ethylenoxid an Fettsäuren, Ester oder Öle.

Die Zusammensetzung kann auch als Gel vorliegen. Zu kosmetischen geeigneten Gelbildnern gehören Carbomer, Xanthangummi, Carrageenan, Akaziengummi, Guargummi, Agar-Agar, Alginate und Tylosen, Carboxymethylcellulose, Hydroxyethylcellulose, quaternisierte Cellulose, quaternisierter Guar, bestimmte Polyacrylate wie Acrylates/C10-30 Alkyl Acrylate Crosspolymer, Polyvinylalkohol, Polyvinylpyrrolidon.

Die Verwendung des erfindungsgemäßen kosmetischen Präparates kann z.B. erfolgen in Sonnencremes, Sonnengelen, After-sun-Produkten, Tagescremes, Nachtcremes, Masken, Körperlotionen, Reinigungsmilch, Grundierungen zur Aufhellung, Körperpuder (mit teilchenförmigem Extrakt), als Anti-Spot-Mittel. Die Herstellung derartiger Produkte erfolgt auf eine Weise, wie sie dem Fachmann auf diesem Gebiet bekannt ist.

Die Erfindung soll nachstehend durch Beispiele näher erläutert werden. Alle Angaben erfolgen in Gewichtsprozent, sofern nichts anderes angegeben ist.

### Beispiel 1 Aufhellungscreme

| **Phase A** | |
|---|---|
| Wasser | q.s. ad 100 |
| Propylenglycol | 5,0 |

| **Phase B** | |
|---|---|
| Steareth-2 | 1,8 |
| Steareth-21 | 2,0 |
| Cetearyl Alcohol | 2,0 |
| Cyclomethicone | 5,5 |

| **Phase C** | |
|---|---|
| **Phase D** | |
| Konservierungsmittel | 0,5 |
| Weiße Orchidee-Extrakt (Wirkstoff* in | |
| Propylenglycol als Extraktionsmittel) | 0,05 |
| Kiwi-Extrakt (ethanolisch) | 1,5 |
| Parfüm | 0,5 |
| Wirkstoffkomplex gemäß Beispiel 1 der | |
| WO99/66881 mit Radikalschutzfaktor | |
| (RPF) von 1925 | 1,0 |

| | |
|---|---|
| * Wirkstoffgehalt ca. 40 Gew-%, bezogen auf das Gewicht des Extraktes von Phalaenopsis amabilis. | |

Die Phasen A und B werden separat unter Rühren und Erwärmen auf 65 °C hergestellt, zusammengegeben und das Gemisch als Phase C 20 Min homogenisiert und abgekühlt auf ca. 35-40 °C. Unter Rühren erfolgt die Zugabe der Phase D.

### Beispiel 2 Hautaufhellungs-Spray

| | |
|---|---|
| Ethanol | 61,5 |
| Wasser | q.s. ad 100 |
| Cyclomethicone | 3,0 |
| Disodium EDTA | 0,01 |
| Glycerin | 4,5 |
| BHT (Di-tert-butylhydroxytoluol) Weiße Orchidee-Extrakt (Wirkstoff* in | 0,05 |
| Ethanol als Extraktionsmittel) Wirkstoffkomplex gemäß Beispiel 1 der | 0,05 |
| WO99/66881 mit Radikalschutzfaktor (RPF) von 1925 | 1,0 |

| | |
|---|---|
| Alle Bestandteile werden bei 20-25 °C homogen verrührt. * wie Beispiel 1 | |

### Beispiel 3 Anti-Spot Hautabdeckung

| **Phase A** | |
|---|---|
| Wasser | q.s. ad 100 |
| Glycerine | 3,0 |
| EDTA | 0,1 |
| Xanthan Gum | 0,8 |
| **Phase B** | |
| Isopropyl Stearate | 2,0 |
| Dicapryl Ether | 2,0 |
| Cetearyl Alcohol | 2,0 |
| Cetyl Alcohol | 4,0 |

| **Phase C** | |
|---|---|
| **Phase D** | |
| Weiße Orchidee-Extrakt (Wirkstoff* in | |
| Propylenglycol als Extraktionsmittel) | 0,5 |
| Wirkstoffkomplex gemäß Beispiel 1 der | |
| WO99/66881 mit Radikalschutzfaktor | |
| (RPF) von 1925 | 1,0 |
| Eisenoxid schwarz | 0,04 |
| Eisenoxid gelb | 0,2 |
| Eisenoxid rot | 0,02 |

| **Phase E** | |
|---|---|
| Konservierungsmittel | 0,5 |
| Parfüm | 0,7 |

| | |
|---|---|
| * wie Beispiel 1 | |

Die Phasen A und B werden separat unter Rühren und Erwärmen auf 70 °C hergestellt, zusammengegeben und bildet die Phase C. Die Phase C wird 20 Min homogenisiert und abgekühlt auf ca. 35-40 °C. Unter Rühren erfolgt nacheinander die Zugabe der Phasen D und E. Danach wird 1 Std. homogenisiert.

### Beispiel 4 Aufhellungspuder (lose) für Gesicht und Körper

| | |
|---|---|
| Talkum | 95,3 |
| Parfüm | 0,5 |
| Weiße Orchidee-Extrakt (in Puderform) | 0,5 |
| Farbstoff weiß (TiO₂) | 0,2 |
| Eisenoxid schwarz | 0,001 |
| Eisenoxid gelb | 0,01 |
| Eisenoxid rot | 0,05 |

Die einzelnen Bestandteile werden nacheinander unter Rühren zusammengegeben und gut miteinander vermischt.

### Beispiel 5 Anwendertest

Es wurde ein Consumer-Test mit 60 Probanden im Alter von 30 bis 72 Jahren durchgeführt. Grundlage für den Test war eine Creme gemäß Beispiel 1 (Formulierung A), sowie allein die Basiscreme mit den Phasen A bis D gemäß Beispiel 1 jedoch ohne den Orchideen- und Kiwi-Extrakt (Formulierung B).

Der Test lief über 4 Wochen und wurde wöchentlich ausgewertet. Die Endauswertung nach 4 Wochen zeigte folgendes Bild.

| | keine leichte mäßige deutliche | | | |
|---|---|---|---|---|
| | Verbesserung der Depigmentierung | | | |
| Formulierung A | 0% | 6% | 27% | 64% |
| Formulierung B | 22% | 73% | 2% | 0% |

Aus dem Test ist klar zu erkennen, dass die Creme mit dem Orchideen-Extrakt der Erfindung bei über 90 % der Probanden einen mäßige bis deutliche Verbesserung der Depigmentierung herbeiführte, also einen deutlichen Aufhellungseffekt zeigte.

## Patentansprüche

1. Kosmetikum zur Depigmentierung, **gekennzeichnet durch** einen Gehalt eines Extraktes aus Blättern, Blüten und Stengeln der weißen Orchidee Phalaenopsis amabilis sowie einen Gehalt an kosmetischen Trägerstoffen, Hilfsstoffen, weiteren Wirkstoffen oder Gemischen davon.

2. Kosmetikum nach Anspruch 1, **dadurch gekennzeichnet, daß** der Anteil des Orchideenextraktes im Bereich von 0,005 bis 5 Gew-% liegt, bezogen auf das Gesamtgewicht des Kosmetikums.

3. Kosmetikum nach Anspruch 2, **dadurch gekennzeichnet, daß** der Anteil des Orchideenextraktes im Bereich von 0,01 bis 1 Gew-% liegt.

4. Kosmetikum nach Anspruch 1, **dadurch gekennzeichnet, daß** es zusätzlich als Wirkstoff Pflanzenextrakte enthält, ausgewählt unter 0,05 bis 0,3 Gew-% eines Extraktgemisches von Süßholz (Glycyrrhiza glabra) und Aspergillusferment in einem Verhältnis 30:1 bis 5:1, 0,001 bis 0,1 Gew-% eines wäßrigen oder wäßrig-alkoholischen Extraktes von Tragant (Astragalus) und Gemischen dieser beiden Extrakte, wobei die Prozentangaben jeweils auf das Gesamtgewicht des Kosmetikums bezogen sind.

5. Kosmetikum nach Anspruch 1, **dadurch gekennzeichnet, daß** es zusätzlich als Wirkstoff einen UVA-Filter, einen UVB-Filter oder ein Gemisch davon enthält.

6. Kosmetikum nach Anspruch 1, **dadurch gekennzeichnet, daß** es zusätzlich einen Kiwi-Extrakt in einer Konzentrationen von 0,5 bis 5 Gew-% enthält, bezogen auf das Gesamtgewicht des Kosmetikums.

7. Kosmetische Verwendung des Kosmetikums nach Anspruch 1 zur Aufhellung von Hautflächen und zur Aufhellung oder Entfernung von dunklen Hautflecken.

## Claims

1. A cosmetic for depigmentation **characterized by** a content of an extract from leaves, flowers and stems of the white orchid Phalaenopsis amabilis and a content of cosmetic carrier substances, auxiliary agents, further active substances, or mixtures thereof.

2. A cosmetic according to Claim 1, wherein the orchid extract ranges from 0.005 to 5% by weight relative to the cosmetic's total weight.

3. A cosmetic according to Claim 2, wherein the orchid extract ranges from 0.01 to 1% by weight.

4. A cosmetic according to Claim 1, wherein said cosmetic contains plant extracts as additional active substance, selected from among 0.05 to 0.3% by weight of an extract mixture obtained from licorice (Glycyrrhiza glabra) and Aspergillus ferment with a ratio ranging between 30:1 and 5:1, 0.001 to 0.1% by weight of an aqueous or aqueous/alcoholic extract from tragacanth (Astragalus), and mixtures of the two aforesaid extracts, the percentages being relative to the cosmetic's total weight.

5. A cosmetic according to Claim 1, wherein said cosmetic contains an UVA filter, an UVB filter or a mixture thereof as additional active substance.

6. A cosmetic according to Claim 1, wherein said cosmetic additionally contains a kiwi extract in a concentration ranging from 0.5 to 5% by weight relative to the cosmetic's total weight.

7. Cosmetic use of the cosmetic according to Claim 1 for whitening skin areas and for whitening or eliminating dark spots on the skin.

## Revendications

1. Agent cosmétique de dépigmentation, **caractérisé par** un contenu d'extraits de feuilles, de fleurs et de tiges d'orchidée blanche *Phalaenopsis amabilis* ainsi que par un contenu de substrats cosmétiques, d'agents auxiliaires, d'autres substances actives ou de mélanges d'entre eux.

2. Agent cosmétique selon la revendication 1, **caractérisé en ce que** la proportion d'extrait d'orchidée est de 0,005 à 5 % en poids, rapportée au poids total de l'agent cosmétique.

3. Agent cosmétique selon la revendication 2, **caractérisé en ce que** la proportion d'extrait d'orchidée est de 0,01 à 1 % en poids.

4. Agent cosmétique selon la revendication 1, **caractérisé en ce qu'**il contient en plus, en tant que substance active, des extraits de plante, sélectionnés parmi 0,05 à 0,3 % en poids d'un mélange d'extraits de réglisse *(Glycyrrhiza glabra*) et de ferment d'aspergillus dans un rapport de 30 : 1 à 5 : 1, 0,001 à 0,1 % en poids d'un extrait aqueux ou aqueux-alcoolique d'adragante *(Astragalus)* et les mélanges de ces deux extraits, les indications de pourcentage étant rapportées à chaque fois au poids total de l'agent cosmétique.

5. Agent cosmétique selon la revendication 1, **caractérisé en ce qu'**il contient en plus, en tant que substance active, un filtre UV-A, un filtre UV-B ou un mélange d'entre eux.

6. Agent cosmétique selon la revendication 1, **caractérisé en ce qu'**il contient en plus un extrait de kiwi, à une concentration de 0,5 à 5 % en poids rapportée au poids total de l'agent cosmétique.

7. Utilisation cosmétique de l'agent cosmétique selon la revendication 1 pour éclaircir les surfaces de la peau et pour éclaircir ou éliminer les taches sombres de la peau.
